# EUROPEAN PATENT APPLICATION

(11) **EP 3 566 644 A1**
(43) Date of publication of application: **13.11.2019**
(21) Application number: 18171395.9
(22) Date of filing: 09.05.2018
(51) Int. Cl.: A61B 5/024, A61B 17/00

(54) **CONTROLLING WEARABLE PATCH ADHESION**

(71) Applicant: Koninklijke Philips N.V., 5656 AE Eindhoven (NL)
(72) Inventor: Vogtmeier, Gereon, 5656 AE Eindhoven (NL); Hakkens, Franciscus Johannes Gerardus, 5656 AE Eindhoven (NL)
(74) Representative: de Haan, Poul Erik

(57) **Abstract**

An electronic device (10, 100, 200) is disclosed comprising a processor (40) adapted to control a patient monitoring system including a wearable patch (10) to be secured to a patient's skin (1) by a curable adhesive, the wearable patch comprising a patient-facing surface (12) comprising a sensor (20) for performing a sensing operation on the patient's body; and at least one UV light source (30) for curing the curable adhesive. The processor (40) is adapted to, when communicatively coupled to the wearable patch, receive a sensing result from the sensor, process the sensing result; and generate a control signal for controlling the at least one UV light source (30) based on the processed sensing result. A patient monitoring system including such an electronic device, a method of operating such an electronic device and a computer program product for such an electronic device are also disclosed.

## Description

### FIELD OF THE INVENTION

The present invention relates to an electronic device comprising a processor adapted to control a patient monitoring system including a wearable patch to be secured to a patient's skin by a curable adhesive, the wearable patch comprising a patient-facing surface comprising a sensor for performing a sensing operation on the patient's body; and at least one UV light source for curing the curable adhesive.

The present invention further relates to a patient monitoring system including such an electronic device.

The present invention further relates to a method of controlling a patient monitoring system with such an electronic device.

The present invention further relates to a computer program product for such an electronic device.

### BACKGROUND OF THE INVENTION

Advances in medical science have led to the advent of new patient monitoring techniques in which the patient wears a patch with an integrated sensor such as an ultrasound transducer-based sensor attached to the patient's skin, which for instance may be used for prolonged hemodynamic monitoring, bladder function monitoring, as well as vascular access monitoring and other monitoring of other (minimally) invasive medical procedures, and so on. This opened up the possibility of continuous monitoring of the patient with minimal discomfort or disruption to the patient.

Such monitoring has in common that the wearable patch typically has to retained in the same position on the skin of the patient for a prolonged period of time in order to safeguard the reliability of the monitoring results. This for example makes the use of non-adhesive contact-improving media such as contact gels in the case of a patch comprising one or more ultrasound sensors less suitable, as an important characteristic of such media is that they allow the sensor to be moved across a patient's body.

For this reason, such wearable patches are commonly secured to the patient, either directly onto the patient's skin or onto an interface layer attached to the patient's skin using an adhesive such that upon application the wearable patch cannot easily move, thereby providing the desired immobilization of the wearable patch. For example, WO 2015/184097 A2 discloses a sweat sensor device for sensing sweat on the skin including one or more sweat sensors and a volume-reducing component that provides a volume-reduced pathway for sweat between the one or more sweat sensors and sweat glands in said skin when the device is positioned on said skin. The volume-reducing component amongst others may include an adhesive with a vertically anisotropic sweat pathway. The adhesive may be a UV-curable adhesive, which may be provided with a UV stimulus by a UV LED array integral to an apparatus used to apply the sweat sensor devices to the skin.

However, the use of such adhesives is also not without problems. For example, prior to initiating the monitoring with such a wearable patch, the patch may need to be repositioned several times, e.g. to achieve a good signal to noise ratio for the sensor signals. In other words, the optimal positioning of the wearable patch on the patient's body may not be straightforward and may require several attempts. However, when using an adhesive to immobilize the wearable patch, the patch may not be easily removed from a sub-optimal skin location for repositioning purposes. This may cause discomfort to the patient and may even damage the wearable patch such that it has to be discarded.

### SUMMARY OF THE INVENTION

The present invention seeks to provide an electronic device comprising a processor adapted to control a patient monitoring system including a wearable patch to be secured to a patient's skin by a curable adhesive such that the curable adhesive is cured in a more intelligent manner.

The present invention further seeks to provide a patient monitoring system including such an electronic device.

The present invention further seeks to provide a method controlling a patient monitoring system with such an electronic device.

The present invention further seeks to provide a computer program product for such an electronic device that implements such a method thereon.

According to an aspect, there is provided an electronic device comprising a processor adapted to control a patient monitoring system including a wearable patch to be secured to a patient's skin by a curable adhesive, the wearable patch comprising a patient-facing surface comprising a sensor for performing a sensing operation on the patient's body; and at least one UV light source for curing the curable adhesive; wherein the processor is adapted to, when communicatively coupled to the wearable patch, receive a sensing result from the sensor, process the sensing result and generate a control signal for controlling the at least one UV light source based on the processed sensing result.

In accordance with the invention, the processor implements a feedback loop in which the sensor result is processed, i.e. evaluated by the processor to determine if a change in the state of the UV light source, e.g. switching from an off state to an on state or from an on state to an off state, is to be effected by generating a control signal for the at least one UV light source. This is based on the insight that the sensor result may be used to evaluate the positioning of the wearable patch on the skin of the patient, as well as to evaluate a degree of curing of the adhesive due to changes in the sensor results with the wearable patch in a stationary location caused by a change in the properties of the adhesive that are the result of the curing process. Consequently, the adhesive for adhering the wearable patch to the patient's skin may be activated by initiating its curing once the wearable patch has been correctly positioned, such that the positioning of the wearable patch may be easily adjusted prior to the initiation of the curing reaction.

In an embodiment, the processor is adapted to generate a control signal to switch on the at least one UV light source upon the processed sensing result meeting a quality requirement. Such a quality requirement for example may be a minimal signal to noise ratio of the sensor signal generated with the sensor.

In another embodiment, the processor is adapted to generate a control signal to switch off the at least one UV light source upon the processed sensing result meeting a further quality requirement. Such a further quality requirement for example may be an image quality requirement in case of an optical or ultrasound sensor, or an interface reflections threshold in case of an interface between the wearable patch and the patient's skin. Alternatively, the cured adhesive layer may define such an interface. This leverages the fact that such adhesives typically have increasing impedance with an increasing degree of curing as the adhesive becomes harder when cured more. Therefore, the further quality requirement may be used to terminate the curing process at the desired degree of curing of the curable adhesive, such as to optimize the adhesion of the wearable patch to the patient's skin and/or to optimize the signal quality of the sensor signals produced by the sensor in the wearable patch. It should be understood that such on/off switching of the at least one light source by the processor may further include modulating the intensity of the luminous output of the at least one UV light source in order to optimize the curing of the adhesive.

The processor further may be adapted to process a series of sensing results and to generate a control signal to switch off the at least one UV light source if the sensing results of said processed series of sensing results exhibit a variance below a defined threshold. In this manner, it may be determined that the curing reaction has completed at least to a desired degree of curing, as towards the end of the curing process the physical properties of the curable adhesive stabilize, which is reflected in a diminishing variance between sensing results in such a series of sensing results such that the monitoring of such variance may be used to terminate the curing process with the at least one UV light source at the desired point in time.

In a further embodiment, the at least one UV light source comprises a plurality of UV light sources each adapted to generate light having a different spectral composition, wherein the processor may be adapted to generate a set of control signals for controlling the plurality of UV light sources in order to tune the spectral composition of the light produced by the plurality of UV light sources.

According to another aspect, there is provide a patient monitoring system comprising a wearable patch to be secured to a patient's skin by a curable adhesive, the wearable patch comprising a patient-facing surface comprising a sensor for performing a sensing operation on the patient's body; at least one UV light source for curing the curable adhesive; and the electronic device of any of the herein described embodiments. Such a patient monitoring system benefits from the aforementioned feedback loop implemented by the electronic device in that the wearable patch can be easily repositioned owing to the fact that the curable adhesive can be intelligently activated once the wearable patch is optimally positioned.

In a preferred embodiment, the electronic device is integral to the wearable patch. In other words, the wearable patch may form a self-contained or stand-alone patient monitoring system in which the processor is integral to the wearable patch.

In the patient monitoring system according to embodiments of the present invention, the at least one UV light source may be integrated in the patient-facing surface of the wearable patch such that the adhesive in between the wearable patch and the patient's skin may be effectively irradiated with UV light. To further improve the effectiveness of the UV irradiation of the entire adhesive layer, the at least one UV light source comprises a plurality of UV light sources distributed across the patient-facing surface. Optionally, the UV light sources are individually addressable, which has the advantage that different regions of the adhesive layer may be cured to a different degree, which for instance may be beneficial to tune regions that are not exposed to the sensor signals to a different degree, e.g. a higher degree of adhesion, than the regions through which the sensor signals travel, which regions may be optimized for transparency to such sensor signals.

In an embodiment, the wearable patch further comprises a light guide across the patient facing surface, said light guide comprising a distribution of light outcoupling structures, wherein the at least one UV light source is arranged to couple UV light into the light guide. This may be beneficial to evenly distribute the UV light across the adhesive layer in between the wearable patch and the patient's skin, thereby obviating the need for a distribution of UV light sources across the patient facing surface of the wearable patch; instead, a single UV light source, which may be integral or external to the wearable patch may be used for this purpose.

The sensor of the wearable patch preferably comprises one or more ultrasound transducers although alternative sensors, e.g. optical sensors, may also be used in the wearable patch according to embodiments of the present invention.

The wearable patch may further comprise a reservoir comprising the curable adhesive. Such a reservoir may be located in between the patient-facing surface of the wearable patch and a removable sealing layer such that the adhesive does not have to be physically transported from the reservoir to the interface between the wearable patch and the patient's skin. Alternatively, the reservoir may be fluidly coupled to such an interface such that physical transport of the adhesive to this interface is required, e.g. using a pump or by manually squeezing the reservoir.

The reservoir may comprise a first chamber comprising a first component of the curable adhesive and a second chamber comprising a second component of the curable adhesive, said first chamber and second chamber being separated by a UV-degradable barrier, and the at least one UV light source includes a UV light source arranged to emit UV light onto said UV-degradable barrier, wherein the processor is arranged to control said UV light source based on the processed sensing result. In this manner, both components of the adhesive are brought together in a controlled manner, e.g. after the wearable patch has been correctly positioned on the patient's skin, thereby further reducing the risk of premature adhesion of the wearable patch to the patient's skin.

Alternatively, such a barrier may be a thermally decomposable barrier such that heating provided by the UV light source decomposes the barrier, or the barrier may be broken by mechanical means, e.g. a needle or the like instead. Such mechanical disruption may be manually achieved by a user or instead the wearable patch may include the mechanical means coupled to an actuator responsive to the processor, such that the processor may generate an actuation signal to cause the mechanical means to puncture or otherwise break the barrier.

Alternatively, the patient monitoring system may further comprise a docking station for the wearable patch, said docking station comprising at least one of the processor and the at least one light source, and further comprising a reservoir comprising the curable adhesive. Such a docking station may be used to facilitate the correct positioning of the wearable patch onto the patient's skin. Moreover, since at least one of the processor and the at least one light source are comprised by the docking station, the power consumption of the wearable patch is reduced, which may increase the battery life of the wearable patch in case of a battery-powered wearable patch.

According to yet another aspect, there is provided a computer-implemented method of controlling a patient monitoring system including a wearable patch to be secured to a patient's skin by a curable adhesive, the wearable patch comprising a patient-facing surface comprising a sensor for performing a sensing operation on the patient's body; and at least one UV light source for curing the curable adhesive; the method comprising receiving a sensing result from the sensor; processing the sensing result; and generating a control signal for controlling the at least one UV light source based on the processed sensing result. Such a method implements a feedback loop in which the sensing results are used as feedback to control the at least one UV light source in order to obtain better control over the curing of the curable adhesive, e.g. to initiate such a curing reaction in response to evidence derived from the sensing result that the wearable patch has been optimally positioned and/or to terminate the curing process in response to a sensing result indicating that a quality requirement for the sensing result has been met.

According to yet another aspect, there is provided a computer program product comprising a computer readable storage medium having computer readable program instructions embodied therewith for, when executed on the processor of the electronic device of any of the herein described embodiments, cause the processor to implement the method according to any of the herein described embodiments. Consequently, an electronic device may be reconfigured or upgraded using such a computer program product to implement the method of the present invention. Such a computer program product may come in the form of an app on a server or the like, or in any other suitable form as will be explained in more detail in the remainder of this application.

### BRIEF DESCRIPTION OF THE DRAWINGS

Embodiments of the invention are described in more detail and by way of non-limiting examples with reference to the accompanying drawings, wherein:
FIG. 1 schematically depicts a cross-sectional view of a wearable patch forming a patient monitoring system according to an embodiment;
FIG. 2 schematically depicts a bottom view of the wearable patch of FIG. 1;
FIG. 3 depicts a flowchart of a computer-implemented method of controlling a patient monitoring system according to embodiments of the present invention;
FIG. 4 schematically depicts a cross-sectional view of a wearable patch forming a patient monitoring system according to another embodiment;
FIG. 5 schematically depicts a cross-sectional view of a wearable patch forming a patient monitoring system according to yet another embodiment;
FIG. 6 schematically depicts an electronic device and a cross-sectional view of a wearable patch forming a patient monitoring system according to yet another embodiment;
FIG. 7 schematically depicts a bottom view of a wearable patch forming at least a part of a patient monitoring system according to yet another embodiment;
FIG. 8 schematically depicts a bottom view of a wearable patch forming at least a part of a patient monitoring system according to yet another embodiment; and
FIG. 9 schematically depicts a cross-sectional view of a wearable patch and a docking station forming a patient monitoring system according to yet another embodiment.

### DETAILED DESCRIPTION OF THE EMBODIMENTS

It should be understood that the Figures are merely schematic and are not drawn to scale. It should also be understood that the same reference numerals are used throughout the Figures to indicate the same or similar parts.

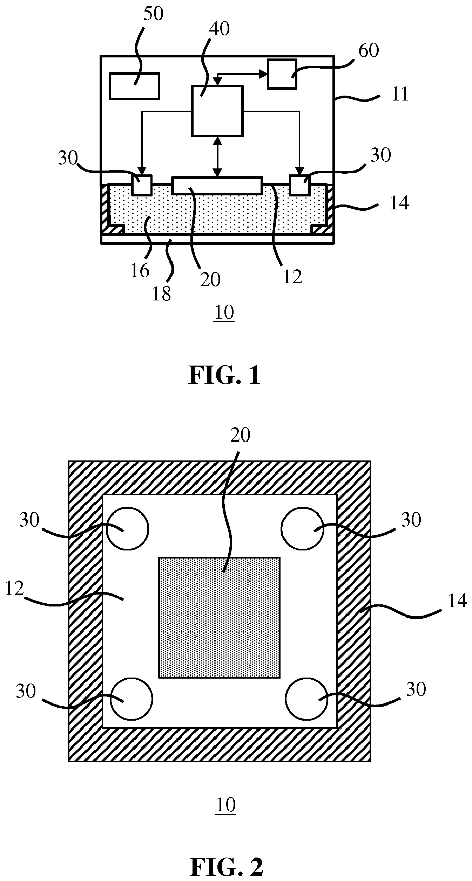

FIG. 1 schematically depicts a wearable patch 10 according to an embodiment of the present invention. The wearable patch 10 comprises a body 11, which may be made of any suitable material, including a major surface 12, which in normal use of the wearable patch 10 faces the skin of the patient. The major surface 12 will therefore also be referred to as the patient-facing surface 12 of the wearable patch 10. The patient-facing surface 12 incorporates a sensor 20 arranged to sense a bodily function of the patient when wearing the wearable patch 10. For example, the sensor 20 may comprise one or more ultrasound transducers arranged to transmit an ultrasound beam into the patient and receive ultrasound echoes of the ultrasound beam for processing, e.g. to image part of the body of the patient. Such ultrasound sensors may be used for several monitoring tasks, such as for example haemodynamic monitoring, e.g. blood flow, pulse pressure, pulse wave velocity, cross-sectional area of arteries, cardiac output and so on, bladder function monitoring in which the rate of urine production of the patient is monitored, visualization of incidents of chronic total inclusion, providing guidance in vascular access procedures, and so on. Other suitable applications of such ultrasound sensors in wearable patches will be immediately apparent to the skilled person. Moreover, it should be understood that embodiments of the present invention are not limited to ultrasound sensors but may refer to any suitable type of sensor 20 that can be used to monitor a patient in such a wearable patch 10.

The patient-facing surface 12 of the wearable patch 10 further comprises at least one UV light source 30, such as a UV LED or another suitable type of light source, which is arranged to irradiate an interface region 16 in between the body 11 of the wearable patch 10 and the skin of the patient with UV light. The interface region 16 may be filled with a UV-curable adhesive that is protected from exposure to an external surface such as the skin of the patient by a removable sealing foil 18, which may be made of any suitable material or combination of materials. Such foils are well-known per se such that this is not explained in further detail for the sake of brevity only. The UV-curable adhesive may be any suitable biocompatible UV-curable adhesive. Non-limiting examples of such adhesives include acrylic-based adhesives such as urethane-acrylates, epoxy-based adhesives, cyanoacrylate-based adhesives and silicones. The UV-curable adhesive may further comprise UV-absorbing particles or dyes to absorb excess UV light and protect the skin of the patient from overexposure to such UV radiation. Such a UV-absorbing agent, e.g. UV-absorbing particles may further be used to create an acoustic impedance gradient across the adhesive layer in case the sensor is an ultrasound transducer, e.g. to create a high acoustic impedance proximal to the ultrasound transducer and a low acoustic impedance proximal to the patient's skin, which for example is advantageous in case the ultrasound transducer comprises one or more PZT elements.

The at least one UV light source 30 in the patient-facing surface 12 of the wearable patch 10 is typically used to initiate the curing of the UV-curable adhesive in the interface region 16. Preferably, a plurality of such UV light sources 30 is distributed across the patient-facing surface 12, e.g. around the one or more sensors 20 as schematically depicted in FIG. 2, or interspersed with such sensors in other to ensure that the interface region 16 is more or less homogeneously illuminated with the UV light to ensure uniform curing of the UV-curable adhesive in order to create uniform bonding of the wearable patch 10 to the skin of the patient by curing the UV-curable adhesive with the UV light emitted by the UV light sources 30.

A sealing ring 14 may be arranged around the interface region 16 to contain the UV-curable adhesive within the interface region 16. The sealing ring 14 preferably is made of an elastomer such that the sealing ring 14 is pliable and upon being brought into contact with a region of the skin of the patient can establish a hermetic seal around the interface region 16, thereby preventing the UV-curable adhesive from overly spreading beyond the interface region 16. Any suitable elastomer, e.g. rubber, may be used for the sealing ring 14. The sealing ring 14 preferably further acts as a UV barrier such that portions of the patient's skin adjacent to the portion onto which the wearable patch is positioned are not exposed to the UV light emitted by the one or more UV light sources 30 in order to protect these skin portions from overexposure to potentially harmful UV radiation. In addition, regions of the patient-surface 12 of the wearable patch 10 may carry wavelength filters that absorb part of the spectrum of the UV light emitted by the one or more UV light sources 30 to limit the light passing through these filters to wavelengths required for the curing of the UV-curable adhesive to further protect the skin of the patient from overexposure to such potentially harmful UV radiation.

The body 11 of the wearable patch 10 further comprises a processor 40 that is communicatively coupled to the sensor 20 and the one or more light sources 30. Such a processor 40 may take any suitable shape, e.g. an ASIC or a generic processor programmed to operate the wearable patch 10 in accordance with embodiments of the present invention as will be explained in further detail below. The processor 40 may be arranged to control operation of the sensor 20 and the one or more UV light sources 30.

The body 11 may further comprise a power source 50 such as a battery in case of a disposable wearable patch 10 or a rechargeable battery in case of a reusable wearable patch 10, which power source 50 is arranged to provide electronic components of the wearable patch 10 such as the sensor 20, the one or more UV light sources 30 and the processor 40. The body 11 of the wearable patch 10 may further comprise a communication interface 60 communicatively coupled to the processor 40, which may take the shape of an access port to which a communication cable may be attached or may take the shape of a wireless communication interface, e.g. a Bluetooth or Wi-Fi interface, or any other suitable wireless communication protocol interface. Such a communication interface 60 may be used to communicate processing results from the processor 40 to an external device such as a bedside monitor, tablet computer or the like onto which the processing results of the processor may be visualized. Alternatively, the wearable patch 10 may further comprise a display (not shown) communicatively coupled to the processor 40, in which case the processor 40 may be arranged to control the display to visualize processing results such as sensing results obtained with the sensor 20.

Alternatively or additionally, the wearable patch 10 may comprise a power supply connection to which the wearable patch 10 may be at least temporarily connected, for example when curing the UV-curable adhesive with the at least one UV light source 30. In this manner, the integrated power source 50 may be protected from the increased power consumption when the at least one UV light source 30 is switched on in order to cure the UV-curable adhesive in order to extend the life of the power supply 50. The power supply connection may be provided through the communication interface 60 or may be a separate power supply connection (not shown).

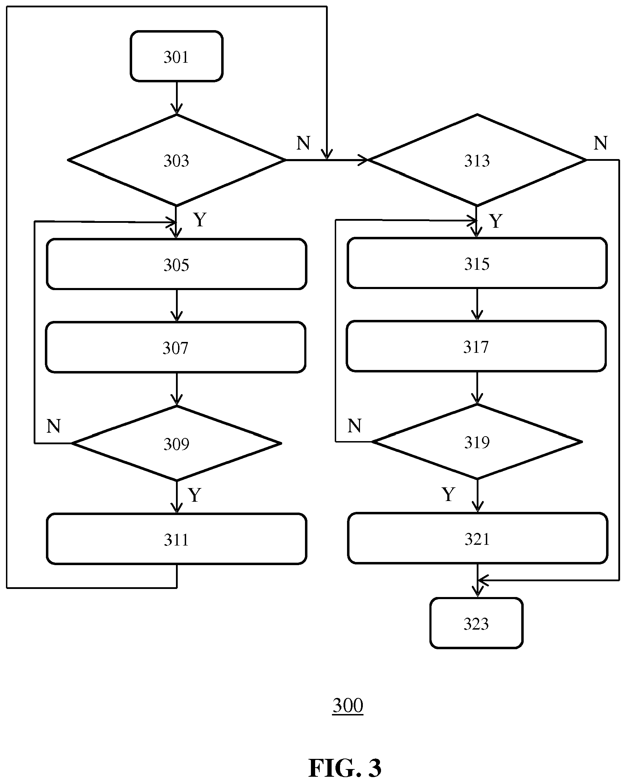

In accordance with embodiments of the present invention, the processor 40 is arranged to assist with the correct positioning of the wearable patch 10 and/or the appropriate degree of curing of the UV-curable adhesive once the wearable patch 10 has been correctly positioned, as will be explained in further detail with the aid of FIG. 3, which depicts a flowchart of a method 300 that may be implemented by the processor 40 in this regard. Generally speaking, the processor 40 is arranged to receive a sensing result from the sensor 20 and the process the sensing result in order to determine a quality indicator of the sensing result, which quality indicator may be linked to the position of the wearable patch 10 on the body of the patient and/or to the degree of curing of the UV-curable adhesive. The processed sensing result including its evaluation is used to determine whether or not the processor 40 is to generate a control signal for enabling or disabling the at least one UV light source 30, thereby effectively implementing a feedback loop in which the processor 40 controls the operation of the sensor 20 and the sensing results obtained with the sensor 20 are being fed back to the processor 40 based on which the processor 40 can make a decision as to the operation of the one or more UV light sources 30.

The method 300 starts in operation 301, e.g. by switching on the processor 40 and starting the positioning process of the wearable patch 10 on the patient's skin in which the wearable patch 10 is placed in an initial position on a portion of the skin of the patient, after which the method 300 proceeds to operation 303 in which it is checked whether the processor 40 should automatically control the at least one UV light source 30 using the aforementioned feedback principle. If this is not the case, the at least one UV light source may be manually enabled or switched on.

However, in a preferred embodiment the processor 40 implements the aforementioned feedback principle, in which case the method 300 proceeds to operation 305 in which the processor 40 controls the sensor 20 to apply a sensing stimulus such as an ultrasound beam to the body of the patient and receives sensing results, e.g. ultrasound echoes, in response to the applied sensing stimulus and processes the received sensing results in operation 307 for example to determine a quality indicator such as a signal-to-noise ratio, a signal strength or the like for the received sensing result. Such a quality indicator can be used to determine whether the wearable patch 10 has been correctly positioned on the patient's skin such that reliable patient monitoring can be performed with the wearable patch 10.

In operation 309, the processor 40 compares the determined quality indicator against a defined quality requirement for the received sensing signal such as a threshold defining an acceptable minimal signal-to-noise ratio or signal strength. If the processor 40 determines that the defined quality requirement has not been met by the received sensing signals, the processor 40 may cause the generation of an audible or visual signal either with the wearable patch 10 or with an external device in communication with the wearable patch 10 to indicate to the person positioning the wearable patch 10 on the skin of the patient that the wearable patch 10 needs to be repositioned in order to improve the quality of the sensing signals, after which the processor 40 reverts back to operation 305. Such an audible or visual signal may be a warning signal or alternatively may be an expression of the determined quality of the sensing signal such that this person can conclude from the expressed quality of the sensing signal, e.g. the displaying of the processed sensing signal on a display of the wearable patch 10 or an external device, that the wearable patch 10 needs to be repositioned. Such feedback assists this person in correctly positioning the wearable patch 10 as will be immediately understood by the skilled person.

On the other hand, if the processor determines in operation 309 that the quality requirement for the received sensing signals has been met, the processor 40 proceeds to operation 311 in which the processor 40 generates a control signal for switching on the at least one UV light source 30 and communicate this control signal directly to the one or more UV light sources 30 or to a controller (not shown) controlling the one or more UV light sources 30.

Where the wearable patch 10 comprises a plurality of UV light sources 30, the processor 40 may generate a global control signal that operates all UV light sources 30 together or alternatively the UV light sources 30 may be individually addressable in which case the processor 40 may control individual control signals for each of the UV light sources 30. This may be advantageous to control the intensity at which the respective UV light sources 30 are being operated, which for example is advantageous in case a multi-layered UV-curable adhesive system is provided such that UV light sources 30 operated at different intensities may effect curing in different layers of the multi-layered UV-curable adhesive system.

Alternatively, different regions of the interface region 16 comprising the UV-curable adhesive may be cured to different degrees in this manner, which for example may be used to optimize adhesive properties in a first region of the interface region 16 and to optimize transmissivity to the sensor signals in a further region of the interface region 16, which further region for example may be directly over the sensor 20. The processor 40 further may be adapted to control the intensity of the UV light produced with the at least one UV light source 30. This for example may be desirable in case particularly high-intensity UV light sources such as UV LEDs are being used that, when operated at full power, may cause overexposure of the patient's skin to UV radiation. In this manner, the processor 40 may control the intensity of the UV light produced with the at least one UV light source 30 to protect the patient's skin from such overexposure.

Alternatively, the UV light sources 30 are arranged to produce luminous outputs having different spectral compositions such that by selectively addressing the UV light sources, e.g. a subset of the UV light sources, the spectral composition of the luminous output of the UV light sources 30 can be accurately controlled.

Next, the method 300 proceeds to operation 313 in which it is checked if the processor 40 needs to deploy the aforementioned feedback loop to control the curing process of the UV-curable adhesive. It is noted that operation 313 may also be reached directly from operation 303 if it is decided in operation 303 that the curing of the UV-curable adhesive is to be initiated manually by manually switching on the one or more UV light sources 30. However, in this scenario the processor 40 will always deploy the aforementioned feedback loop to control the curing process of the UV-curable adhesive as will be described in further detail below.

If the curing process was initiated by the processor 40 as previously explained, the processor 40 may generate a further control signal after a set period of time to switch off the one or more UV light sources 30 engaged in the curing of the UV-curable adhesive. This set period of time is defined to obtain an acceptable degree of curing of the UV-curable adhesive such that the wearable patch 10 may be effectively adhered to the skin of the patient whilst at the same time allowing transmission of the sensor signals between the sensor 20 and the patient's body, after which the method 300 terminates in operation 323. Alternatively, the one or more UV light sources 30 may be manually switched off, e.g. once it has been established that the wearable patch 10 is sufficiently adhered to the skin of the patient or to an interface layer between the patient's skin and the wearable patch 10. Such an interface layer for example may be deployed to facilitate removal of the wearable patch 10 from the skin of the patient after use.

However, in a preferred embodiment the processor 40 is configured to adaptively control the curing process of the UV-curable adhesive based on the received sensing results from the sensor 20. In this embodiment, the processor 40 proceeds to operation 315 in which the processor 40 causes the sensor 20 to deploy a sensor signal to the patient's body and receives the sensing results, i.e. the responses to the deployed sensor signal, from the sensor 20 as previously explained, after which the processor 40 processes the received sensing results in operation 317 for example to determine a further quality indicator such as a signal-to-noise ratio, a degree of signal scattering or the like for the received sensing result. In another embodiment, the further quality indicator may be a known good measurement result, such that upon detecting this measurement result, this may be used as an indicator that the adhesive has sufficiently cured. Such a known good measurement result may be obtained in any suitable manner, e.g. from knowledge obtained with other, previously applied, wearable patches, through artificial intelligence, and so on.

Such a further quality indicator can be used to determine whether the UV-curable adhesive has cured to a sufficient degree, as the acoustic impedance of the UV-curable adhesive will be more closely matched to that of the sensor 20 with increased curing as the UV-curable adhesive becomes harder and its acoustic impedance increases, which improves the signal-to-noise ratio of the sensing results and reduces signal scattering by poorly matched acoustic impedances. As an alternative, the processor 40 may be arranged to process a series of sensing results to determine if the sensing results of said processed series of sensing results exhibit a variance below a defined threshold. This may be deployed to determine whether the curing of the UV-curable adhesive nears completion, as upon completion of the curing of the UV-curable adhesive the variation between successive sensing results will diminish as the physical properties such as the acoustic impedance of the UV-curable adhesive stabilize, which may be another suitable metric to determine whether the curing process of the UV-curable adhesive may be terminated.

In operation 319, the processor 40 compares the determined further quality indicator for the variance between successive sensing results against a defined threshold such as a defined further quality requirement for the received sensing signal such as a threshold defining an acceptable minimal signal-to-noise ratio or a maximum signal reflection threshold, or a threshold defining a maximum acceptable variance between successive sensing results. If the processor 40 determines that the defined further quality requirement has not been met by the received sensing signals, the processor 40 reverts back to operation 315. Otherwise, the processor 40 proceeds to operation 321 in which the processor generates a further control signal to be deployed directly to the one or more UV light sources 30 or to a controller of these light sources that causes the one or more UV light sources 30 to be switched off, after which the method 300 terminates in operation 323 as previously explained.

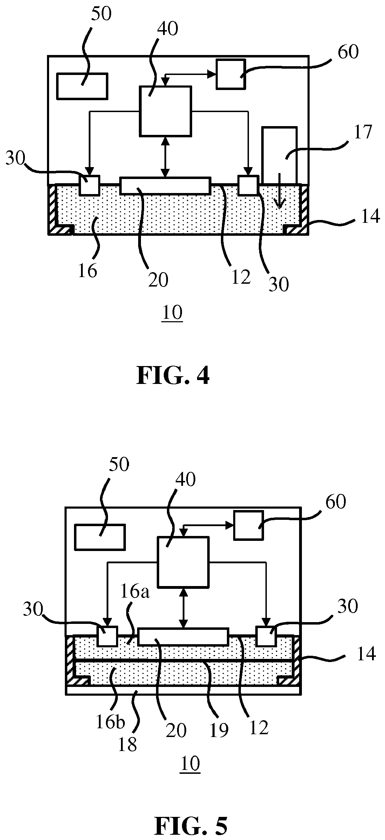

In FIG. 1, the UV-curable adhesive was contained in the reservoir defined by the interface region 16 in between the body 11 of the wearable patch 10 and the sealing foil 19, which has the advantage that the UV-curable adhesive is already located in the correct location for initiating the adhesion between the wearable patch 10 and the skin of the patient. However, it should be understood that this reservoir may be situated in any suitable location within the wearable patch 10 as is schematically depicted by way of non-limiting example in FIG. 4, in which the reservoir 17 containing the UV-curable adhesive is located within the body 11 of the wearable patch 10. The reservoir 17 typically is in fluid collocation with the interface region 16 such that upon correct positioning of the wearable patch 10 on the skin of the patient the UV-curable adhesive may be transported from the reservoir 17 to the interface region 16. This may be achieved in any suitable manner, for example by a user of the wearable patch 10 compressing the reservoir 17 to expel the UV curable adhesive into the interface region 16 or by means of an integrated pump (not shown) under control of the controller 40 that pumps the UV-curable adhesive into the interface region 16.

In yet another embodiment, the curable adhesive may be a multi-component adhesive system in which the curing may be initiated once the various components of the adhesive system have been brought into contact with each other. To this end, as is schematically depicted in FIG. 5, the wearable patch 10 may comprise multiple reservoirs here denoted 16a and 16b that are separated by a barrier 19. The barrier 19 may be a UV-degradable or thermally degradable barrier such that upon correct positioning of the wearable patch 10 on the skin of the patient the controller 40 switches on the one or more UV light sources 30 as previously explained, which causes the degradation of this barrier 19 allowing the various components of the adhesive system to contact each other such that the curing process may be initiated. It is noted that in this embodiment the curing process of the adhesive does not necessarily require UV radiation; however, UV radiation is required to establish contact between the various components of the adhesive by degradation of the degradable barrier 19 by means of the one or more UV light sources 30. Alternatively, the wearable patch 10 may comprise an actuated mechanical piercing member (not shown) responsive to the processor 40 such that the processor can actuate this member at the appropriate point in time as explained in more detail above to disrupt the barrier 19 and allow the various components of the adhesive system to contact each other. The metal piercing member alternatively may be activated through heating with the UV light source, e.g. may take the shape of a bimetal or the like.

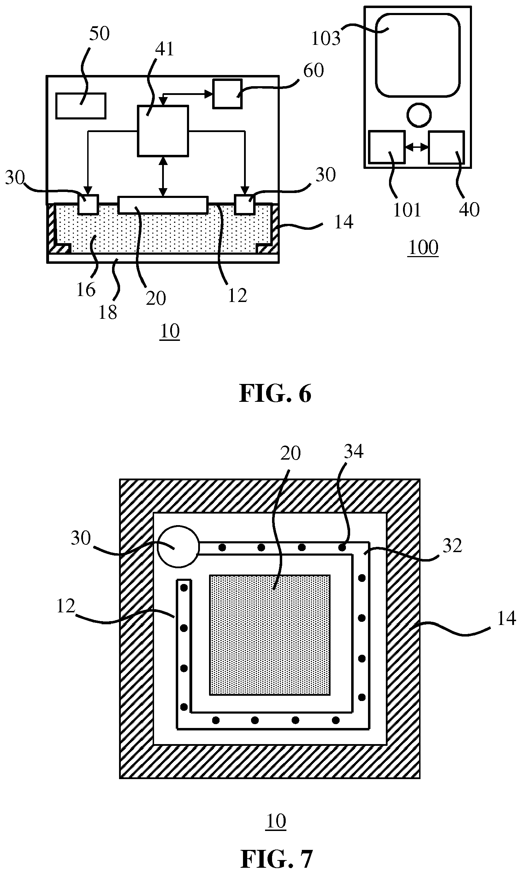

In order to extend the life of the power supply 50 of the wearable patch 10, at least some of the more computationally intensive signal processing may be performed on an external device. This is schematically depicted in FIG. 6, in which the wearable patch 10 is communicatively coupled to an external device 100 through its communication interface 60 and the communication interface 101 of the external device 100. The external device 100 may further comprise a user interface 103 such as a touchscreen or the like through which a user of the external device 100 can operate the wearable patch 10 and/or can evaluate sensor results obtained with the external device 100. For example, the wearable patch 10 and the external device 100 may communicate wirelessly using any suitable wireless communication protocol such as Wi-Fi, Bluetooth, a mobile communication protocol such as GSM, UMTS, a proprietary communication protocol and so on. The external device 100 is depicted as a smart phone by way of non-limiting example only. Although the external device 100 may be a smart phone or another portable device such as a smart watch or a tablet computer, the external device 100 alternatively may be a central server, computer, bedside monitor or the like, which for example may be advantageous in a medical facility such as a hospital to centrally monitor the patient wearing the wearable device 10.

The processing of the sensing results acquired with the sensor 20 may be performed on the external device 100. To this end, the external device 100 may comprise the processor 40, in which case the wearable device 10 comprises a further processor or controller 41, which may be configured to perform some pre-processing of the sensing signals, e.g. down conversion or the like, in order to reduce the amount of data that needs to be communicated to the external device 100, thereby further extending the life of the power supply 50, e.g. a battery. In this embodiment, the implementation of the feedback loop may at least in part be performed by the processor 40 on the external device 100 including the processing of the received sensing results and the generation of the control signal based on these processed sensing results for the one or more UV light sources 30, which may be communicated to the wearable patch 10 over its communication link with the external device 100.

As previously explained, in order to achieve a substantially homogeneous irradiation of the interface region 16 with UV light, a plurality of UV light sources 30 may be deployed distributed across the patient-facing surface 12 of the wearable patch 10. Alternatively, as schematically depicted in FIG. 7, the wearable patch 10 may further comprise a light guide 32 such as a glass fibre or the like on the patient-facing surface 12 and arranged around the sensor 20 in order to such a substantially homogeneous irradiation of the interface region 16 with UV light. To this end, the light guide 32 may include a plurality of outcoupling structures 34, which may be arranged in a regular pattern or the like such that at regular intervals UV light coupled into the light guide 32 is coupled out towards the interface region 16 by such outcoupling structures 34. Any suitable outcoupling structure, e.g. white paint dots or the like, may be used for this purpose. A benefit of using such a light guide 32 is that fewer UV light sources 30 need to be integrated into the wearable patch 10 such that the life of the power supply 50 may be extended owing to the fact that less energy is consumed during the curing of the UV-curable adhesive in the interface region 16. This is symbolically depicted in FIG. 7 by the patient-facing surface 12 of the wearable patch 10 comprising a single UV light source 30 only, which is arranged to couple its luminous output into the light guide 32.

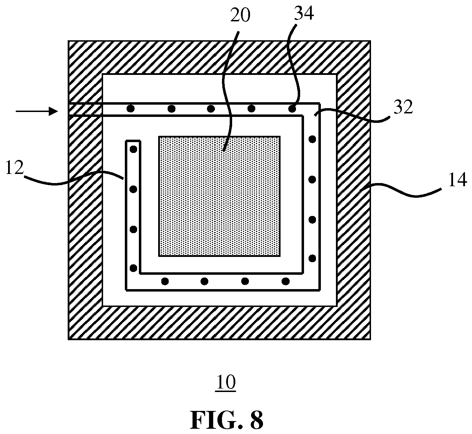

Alternatively, as schematically depicted in FIG. 8, the light guide 32 may extend to the periphery of the wearable patch 10, e.g. through the elastomer seal 14, such that the UV light may be provided by a UV light source external to the wearable patch 10, which has the advantage that the wearable patch 10 does not need to include the UV light source at all, thereby reducing manufacturing cost of the wearable patch 10 and further increasing the life of the power supply 50 as its power does not need to be used to operate the UV light source. It is noted for the avoidance of doubt that in this embodiment such an external UV light source is still controllable by the processor 40, for example through a communication link between the device comprising the processor 40 and a controller of the external UV light source such that the aforementioned feedback principle used to engage or disengage UV light source in the curing process of the UV-curable adhesive in the interface region 16 can still be deployed.

In the aforementioned embodiments of a patient monitoring system according to the present invention, the patient monitoring system has been embodied by a wearable patch 10 in isolation, in which case the wearable patch 10 comprises both the processor 40 and one or more UV light sources 30. Alternatively, the patient monitoring system has been embodied by wearable patch 10 and an external device 100, in which the processor 40 is located within the external device 100. In a further variant, the patient monitoring system is embodied by such a wearable patch 10, optionally an external device 100 and at least one UV light source 30 external to the wearable patch 10 under control of the processor 40, which external UV light source 30 is typically arranged to irradiate the interface region 16, either directly or through use of a light guide 32 as previously explained.

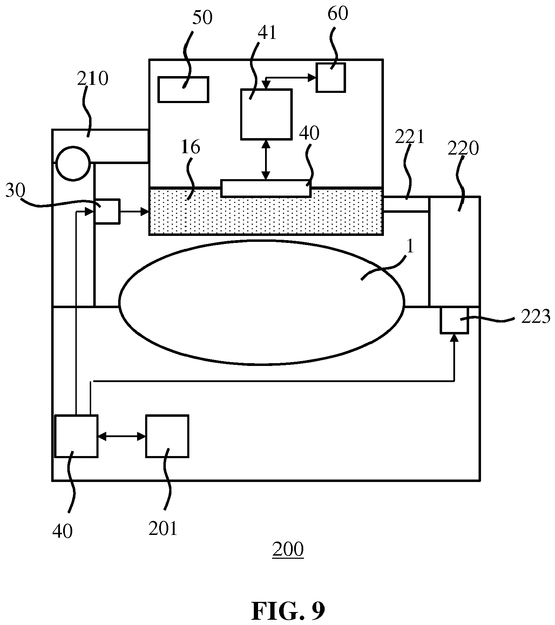

In yet another embodiment, which is schematically depicted in FIG. 9, the patient monitoring system may further comprise a docking station 200, which may be shaped to receive a body portion 1 of the patient such as the patient's arm or leg. The docking station 200 comprises an adjustable holder 210 adapted to hold the wearable patch 10 in position relative to the body portion 1 of the patient to assist with the correct positioning of the wearable patch 10 onto the body portion 1. The docking station 200 may further comprise an external reservoir 220 containing the UV-curable adhesive, which may be fluidly coupled to the interface region 16 through a conduit 221 such as a pipe, a length of tubing, or the like. The UV-curable adhesive may be manually expelled from the external reservoir 220, e.g. by compressing the external reservoir 220 or by operating a manual pump (not shown) between the external reservoir 220 and the conduit 221 that forces the UV-curable adhesive from the external reservoir 220 into the interface region 16. Alternatively, the docking station 200 may comprise a motorised pump 223 under control of the processor 40, in which case the processor 40 may be adapted to enable the motorised pump 223 upon determining that the wearable patch 40 has been correctly positioned onto the body portion 1 of the patient as previously explained, such that upon the determination of such correct positioning the motorised pump 223 forces the UV-curable adhesive from the external reservoir 220 into the interface region 16.

The processor 40 may be located within the wearable patch 10 as previously explained. Alternatively, the docking station 200 may comprise the processor 40 to extend the life of the power supply 50 for the same reasons as previously explained in the embodiment in which the processor 40 was located within the external device 100. To this end, the docking station 200 may further comprise a communication interface 201 that facilitates communication between the processor 40 and the controller 41 within the wearable patch 10 over a communications link established between the communication interface 201 and the communication interface 60 of the wearable patch 10. In this embodiment, the feedback loop as described above in more detail is therefore implemented on the docking station 200 by its processor 40.

The one or more UV light sources 30 may be located within the wearable patch 10 as previously explained. Alternatively, the docking station 200 may comprise the one or more UV light sources 30 under control of the processor 40, which one or more UV light sources 30 may be arranged on the docking station 200 such that their luminous output may be directly aimed at the interface region 16 or may be provided to the interface region 16 using the light guide 32 on the patient-facing surface 12 of the wearable patch 10 as previously explained. The one or more UV light sources 30 may be arranged on the docking station 200 in any suitable location, such as on the adjustable holder 210 by way of non-limiting example.

The above described embodiments of the method 300 may be realized by computer readable program instructions embodied on a computer readable storage medium having, when executed on the processor 40 of the patient monitoring system, cause the processor 40 to implement the method 300. Any suitable computer readable storage medium may be used for this purpose, such as for example an optically readable medium such as a CD, DVD or Blu-Ray disc, a magnetically readable medium such as a hard disk, an electronic data storage device such as a memory stick or the like, and so on. The computer readable storage medium may be a medium that is accessible over a network such as the Internet, such that the computer readable program instructions may be accessed over the network. For example, the computer readable storage medium may be a network-attached storage device, a storage area network, cloud storage or the like.

The computer readable storage medium may be an Internet-accessible service from which the computer readable program instructions may be obtained. In an embodiment, the patient monitoring system is adapted to retrieve the computer readable program instructions from such a computer readable storage medium and to create a new computer readable storage medium by storing the retrieved computer readable program instructions in a data storage arrangement, e.g. in a memory device or the like forming part of the data storage arrangement, which data storage arrangement is accessible to the processor 40 of the patient monitoring system such that the processor can retrieve the computer-readable program instructions from the data storage arrangement for execution.

The embodiments of the present invention have been described in the context of a wearable patch 10 that typically is used in a medical context, e.g. patient monitoring. However, it should be understood that the teachings of the present invention may also be deployed in other technical fields. Generally speaking, the teachings of the present invention may be deployed in any technical field, such as industrial applications, in which a patch is to be attached to a surface, and in which the adhesion of the patch to that surface may be controlled using a sensor within the patch that provides a processor with sensing results such that the processor can monitor the degree of curing of the adhesive as triggered by a source of UV radiation by processing the sensing results and controlling the source of UV radiation based on the processed sensing results as explained in more detail above. It should be understood by the skilled person that any of the above described embodiments equally may be deployed in such other application domains by adjusting the functionality of the patch accordingly. This for example may include a adjusting the type of UV-curable adhesive used, the size of the patch, the sensor technology used within the patch, and so on. Notwithstanding such changes, the skilled person will understand that the feedback principle in which a processor receives sensing results from a sensor in such a patch that are affected by the degree of curing of the UV-curable adhesive and determines the degree of curing of the adhesive by processing the received sensing results in order to disable one or more UV light sources once the desired degree of curing has been achieved is not particularly limited to the medical field and therefore may be deployed in any (industrial) application.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. The word "comprising" does not exclude the presence of elements or steps other than those listed in a claim. The word "a" or "an" preceding an element does not exclude the presence of a plurality of such elements. The invention can be implemented by means of hardware comprising several distinct elements. In the device claim enumerating several means, several of these means can be embodied by one and the same item of hardware. The mere fact that certain measures are recited in mutually different dependent claims does not indicate that a combination of these measures cannot be used to advantage.

## Claims

1. An electronic device (10, 100, 200) comprising a processor (40) adapted to control a patient monitoring system including:
a wearable patch (10) to be secured to a patient's skin (1) by a curable adhesive, the wearable patch comprising a patient-facing surface (12) comprising a sensor (20) for performing a sensing operation on the patient's body; and
at least one UV light source (30) for curing the curable adhesive;
wherein the processor is adapted to, when communicatively coupled to the wearable patch:
receive a sensing result from the sensor;
process the sensing result; and
generate a control signal for controlling the at least one UV light source based on the processed sensing result.

2. The electronic device (10, 100, 200) of claim 1, wherein the processor (40) is adapted to generate a control signal to switch on the at least one UV light source (30) upon the processed sensing result meeting a quality requirement.

3. The electronic device (10, 100, 200) of claim 1 or 2, wherein the processor (40) is adapted to generate a control signal to switch off the at least one UV light source (30) upon the processed sensing result meeting a further quality requirement.

4. The electronic device (10, 100, 200) of claim 3, wherein the processor (40) is adapted to process a series of sensing results and to generate a control signal to switch off the at least one UV light source (30) if the sensing results of said processed series of sensing results exhibit a variance below a defined threshold.

5. A patient monitoring system comprising:
a wearable patch (10) to be secured to a patient's skin by a curable adhesive, the wearable patch comprising a patient-facing surface (12) comprising a sensor (20) for performing a sensing operation on the patient's body;
at least one UV light source (30) for curing the curable adhesive; and
the electronic device (10, 100, 200) of any of claims 1-4.

6. The patient monitoring system of claim 5, wherein the electronic device is integral to the wearable patch (10).

7. The patient monitoring system of claim 5 or 6, wherein the at least one UV light source (30) is integrated in the patient-facing surface (12) of the wearable patch (10).

8. The patient monitoring system of claim 7, wherein the at least one UV light source (30) comprises a plurality of UV light sources distributed across the patient-facing surface (12), optionally wherein the UV light sources are individually addressable.

9. The patient monitoring system of any of claims 5-8, further comprising a light guide (32) across the patient monitoring surface comprising a distribution of light outcoupling structures (34), wherein the at least one UV light source (30) is arranged to couple UV light into the light guide.

10. The patient monitoring system of any of claims 5-9, wherein the sensor (20) comprises an ultrasound transducer.

11. The patient monitoring system of any of claims 5-10, wherein the wearable patch (10) further comprises a reservoir (16, 17) comprising the curable adhesive.

12. The patient monitoring system of claim 11, wherein the reservoir comprises a first chamber (16a) comprising a first component of the curable adhesive and a second chamber (16b) comprising a second component of the curable adhesive, said first chamber and second chamber being separated by a UV-degradable barrier (19), and the at least one UV light source (30) includes a UV light source arranged to emit UV light onto said UV-degradable barrier, wherein the processor (40) is arranged to control said UV light source based on the processed sensing result.

13. The patient monitoring system of claim 5, further comprising a docking station (200) for the wearable patch (10), said docking station comprising at least one of the processor (40) and the at least one light source (30), and further comprising a reservoir (22) comprising the curable adhesive.

14. A computer-implemented method (300) of controlling a patient monitoring system including:
a wearable patch (10) to be secured to a patient's skin (1) by a curable adhesive, the wearable patch comprising a patient-facing surface (12) comprising a sensor (20) for performing a sensing operation on the patient's body; and
at least one UV light source (30) for curing the curable adhesive;
the method comprising:
receiving (305, 315) a sensing result from the sensor;
processing (307, 317) the sensing result; and
generating (311, 321) a control signal for controlling the at least one UV light source based on the processed sensing result.

15. A computer program product comprising a computer readable storage medium having computer readable program instructions embodied therewith for, when executed on the processor (40) of the electronic device (10, 100, 200) of any of claims 1-4, cause the processor arrangement to implement the method (300) of claim 14.
